# EUROPEAN PATENT APPLICATION

(11) **EP 4 670 771 A1**
(43) Date of publication of application: **31.12.2025**
(21) Application number: 24184305.1
(22) Date of filing: 25.06.2024
(51) Int. Cl.: A61M 25/00

(54) **URINARY CATHETER ASSEMBLY**

(71) Applicant: Wellspect AB, 431 21 Mölndal (SE)
(72) Inventor: LOVMAR, Martin, 431 69 MÖLNDAL (SE); HOOFT GRAAFLAND, Joy, 411 02 Göteborg (SE)
(74) Representative: Aldridge, Henry Alexander

(57) **Abstract**

The present disclosure relates to a urinary catheter assembly and method for using the assembly. The assembly comprises a container and a urinary catheter comprising a catheter shaft with a central lumen and a holder arrangement attached to a distal region of the catheter shaft. The catheter shaft is provided with at least one drainage opening communicating with the central lumen and the holder arrangement comprises a distal end cavity communicating with the central lumen. The assembly further comprises a lid and a sealing arrangement configured to form a liquid and/or gas tight seal between the holder arrangement and the container when the urinary catheter is inserted into the container, wherein the sealing arrangement is further configured to allow the urinary catheter to be displaced in the axial direction while maintaining the liquid and/or gas tight seal.

## Description

### TECHNICAL FIELD OF THE INVENTION

The present invention relates to a urinary catheter assembly, in particular a reusable urinary catheter, and methods for using the urinary catheter assembly.

### BACKGROUND OF THE INVENTION

Various types of urinary catheters are used to empty urine from the urine bladder by inserting the urinary catheter through the user's urethra or a stoma. There are mainly two types of urinary catheters: intermittent catheters and indwelling catheters. Indwelling catheters (also known as Foley catheters) are adapted for long time use whereby the indwelling catheters are typically configured to remain in the bladder for a long period of time, such as several hours, the majority of a day or even longer, allowing urine to continuously drain from the bladder.

Intermittent urinary catheters, on the other hand, are generally configured for intermittent use during much shorter periods of time and typically meant to be used only once, to empty the bladder and subsequently discarded. Modern intermittent catheters are becoming more and more comfortable to use, are provided in more convenient packages and are well suited for self-catheterization whereby intermittent single-use urinary catheters are used extensively in both hospitals and in home-settings.

A drawback with single-use intermittent urinary catheters is that they produce a considerable amount of waste. For example, single use intermittent urinary catheters are typically packaged individually in sterile packages, and after the package has been opened and the catheter used to drain the bladder of the user, both the package and the catheter are discarded. Since intermittent catheterization is performed by each user multiple times each day, with a new intermittent urinary catheter being used each time, it follows that a single user may produce thousands of single-use intermittent urinary catheters and associated packaging as waste each year.

To reduce the amount of waste and provide a more sustainable alternative to single use intermittent urinary catheters, various types of multiple-use intermittent urinary catheters have been proposed.

In some multiple-use solutions a reusable urinary catheter is put inside a closable case after each use, wherein a disinfection liquid is poured into the case so as to disinfect the urinary catheter between uses. Another flavor of multiple-use solutions instead relies on a case provided with an internal UV light emitting element whereby the UV light is used to disinfect the urinary catheter between uses.

However, a drawback with the existing multiple-use solutions for intermittent urinary catheters is that it generally is difficult to ensure that the disinfecting liquid or UV light properly disinfects all parts of the urinary catheter that need disinfection between subsequent uses. An improperly disinfected urinary catheter may lead to infections such as urinary tract infections, UTIs. Additionally, while placing a urinary catheter inside a case together with an excessive amount of disinfection liquid may generally achieve satisfactory disinfection, the catheter is completely wetted by disinfection liquid upon removal making it difficult to grip and handle.

Accordingly, there is a need for an improved urinary catheter arrangement that is convenient to use and which achieves sufficient disinfection between subsequent uses.

### SUMMARY OF THE INVENTION

It is a purpose of the present disclosure to present an improved urinary catheter assembly, configured to allow a liquid agent (e.g. for disinfection, regeneration and/or wetting) to reach all critical surfaces of the urinary catheter. This may e.g. allow the urinary catheter to be reused multiple times.

According to an aspect of the present invention there is provided a urinary catheter assembly comprising a container extending at least along an axial direction, from an open first portion to an opposite closed second portion. The assembly further comprises a urinary catheter comprising a catheter shaft with a central lumen and a holder arrangement attached to a distal region of the catheter shaft, wherein the catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen. The assembly further comprises a sealing arrangement configured to form a liquid and/or gas tight seal between the holder arrangement and the container when the urinary catheter is inserted into the container, wherein the sealing arrangement is further configured to allow the urinary catheter to be displaced in the axial direction while maintaining the liquid and/or gas tight seal and a lid, configured to seal the distal end cavity of the holder arrangement.

The present invention is at least partially based on the understanding that by providing a sealing arrangement which forms a liquid and/or gas tight seal between the container and the holder arrangement, and which allows the urinary catheter to be displaced in the axial direction while still maintaining the liquid and/or gas tight seal, the urinary catheter will act as a piston. More specifically, the urinary catheter's insertion into the container increases the pressure inside the container whereby the increase in pressure will drive any liquid agent provided in the container into the drainage openings, up through the central lumen of the catheter and into the distal end cavity, thereby reaching all critical surfaces in need of disinfection and/or rinsing.

Hereby, the urinary catheter assembly of the present invention can achieve excellent disinfection and the assembly may be used to repeatedly disinfect a reusable urinary catheter allowing reuse of the catheter while reducing waste. However, it is also envisaged that the assembly may be used generally to wet, regenerate and/or disinfect a urinary catheter with the liquid agent regardless of the urinary catheter being intended for reuse or single time use.

Additionally, the effect of driving the liquid agent through the central lumen via the drainage openings is achieved for a wide range of liquid agent volumes inside the container. Notably, as the amount of liquid agent decreases between uses the container contains progressively more air which may still aid in providing a pressure driving the remaining liquid agent into the central lumen.

Yet another advantage is that a reverse negative pressure effect will occur when the urinary catheter is removed, actively withdrawing any liquid agent in the distal end cavity and central lumen into the container, whereby the urinary catheter upon removal from the container does not drip or overflow of liquid agent.

The first portion of the container comprises a container opening and when the urinary catheter is inserted into the container a proximal tip of the urinary catheter first passes through the container opening into the first portion of the container and the proximal tip subsequently reaches the second portion of the container. Then, the holder arrangement reaches the first portion of the container, and the sealing arrangement forms the liquid and/or gas tight seal generally in the first portion of the container, trapping the liquid agent in the second portion of the container. Accordingly, the first portion of the container is for forming the liquid and/or gas tight seal and the second portion of the container is generally for storing the catheter shaft and the liquid agent.

The liquid agent is a liquid for achieving at least one of wetting, disinfection and regeneration of a hydrophilic coating on the catheter. For example, the liquid agent is a disinfecting agent or a combined disinfecting and wetting agent.

In some implementations, the sealing arrangement comprises a sealing element arranged on at least one of the holder arrangement and the container.

For example, the sealing element is arranged on an inner surface of the first portion of the container or on an outer surface of the holder arrangement.

The sealing element will accordingly not be in the way during catheterization as the sealing element is located on the holder arrangement and/or in the container whereby the insertable proximal portion of the catheter shaft remains free when the catheter is removed from the container. Optionally, multiple cooperating sealing elements are provided to further facilitate a reliable sealing. For example, sealing elements may be arranged on both the holder arrangement and on the inside the first portion of the container.

In some implementations, the sealing element is realized using a piece of compressible or flexible material and/or a piece of material with shape memory. A benefit of using a compressible, flexible or shape-memory material is that the shape and size of the sealing element can easily be tailored after the dimensions of the holder arrangement and/or the container while enabling a liquid and/or gas tight seal to be formed.

In some implementations, the sealing element comprises a gasket.

For example, the gasket is provided as a cuff surrounding the holder arrangement or the inside of the first portion of the container.

Additionally or alternatively, the sealing element comprises a sleeve made of a flexible material, wherein the sleeve comprises a narrow end attached to the holder arrangement and a wide end, wherein at least one of the narrow end and the wide end is configured to form the liquid and/or gas tight seal against the container.

A benefit with a sleeve shaped sealing element is that the urinary catheter can be displaced along the axial direction, while maintaining the liquid and/or gas tight seal, without requiring a sealing element which slides against the container. Sliding engagement may cause wear on the sealing element which reduces the expected lifetime of the sealing arrangement. Additionally, the sleeve can be turned inside out providing a comparatively large dry surface for a user to grip while also allowing the liquid agent to contact substantially the entire catheter shaft when the catheter is inserted into the container.

In some implementations, the holder arrangement comprises a connector part fixedly attached to the catheter shaft and a handling part configured to be releasably connected to the connector part.

The handling part is further configured to slide over the catheter shaft after it has been released from the connector part. Accordingly, the holder arrangement may be realized as a two-piece arrangement comprising a releasable handling part and a connector part fixedly attached to the catheter shaft. With the handling part the urinary catheter may be easier to use since users can hold the urinary catheter by the connector part and by the handling part (which is slidable over the catheter) without coming into direct contact with the catheter shaft. Generally, contacting the catheter shaft prior to or after catheterization is undesirable, primarily since the catheter shaft should be kept sterile prior to catheterization.

In some implementations, the holder arrangement is a single piece arrangement whereby the single piece holder arrangement is fixedly attached to the catheter shaft.

In some implementations, a sealing element forming part of the sealing arrangement is provided on the handling part.

A benefit with providing the sealing element on the handling part is that at least a portion of the handling part may hereby be kept out of contact with the liquid agent whereby the handling part is kept dry to the touch making it easier and more pleasant for users to grip. A further benefit by providing the sealing element on the handling part is that the connector part could be similar or partially identical to conventional connectors for urinary catheters. Hereby, users will be familiar with the look and feel of the connector part and can connect conventional extension tubes and/or urine collection bags to the connector part.

In some implementations, the holder arrangement is configured for threaded engagement with the first portion of the container.

Accordingly, each of the holder arrangement and the first portion of the container may be provided with a matching set of threads, whereby the holder arrangement can be threaded into the first portion of the container. The threaded engagement may be used in addition to the sealing arrangement, or the threaded engagement may serve as the sealing arrangement.

A benefit of utilizing threaded engagement between the holder arrangement of the urinary catheter and the container is that the urinary catheter is kept firmly inside the container in the storage state. Additionally, threaded engagement may limit the speed at which the urinary catheter is inserted axially into the container, which is advantageous since it reduces the risk of too rapid insertion whereby liquid agent could be ejected from the container and/or distal end cavity of the urinary catheter. Additionally, threaded engagement allows the axial displacement of the catheter relative the container to be accurately defined, which in turn allows the displacement volume during insertion of the catheter to be defined accurately to e.g. prohibit overflow and ensure that liquid agent reaches the distal end cavity.

As mentioned above, threaded engagement may also serve as the sealing arrangement. Hereby, in addition or as an alternative to sealing elements comprising a piece of flexible or compressible material and/or a piece of material with shape memory described above, a sealing arrangement realized by threaded engagement between the container and holder arrangement part is another option.

In some implementations, the distance along the axial direction which the urinary catheter can be displaced while the liquid and/or gas tight seal is maintained is configured such that a volume inside the container changes with a difference volume being at least a volume defined by the central lumen.

The volume of the central lumen may be defined as the cross-sectional area of the central lumen multiplied with the length of the lumen. If the central lumen has a varying cross-sectional area the volume of the central lumen may be defined by integrating the cross-sectional of infinitesimal thick sections along the central lumen. The volume of the central lumen can also be determined experimentally, for example by closing the eyelets and determining the amount of liquid that can be stored inside the central lumen.

If the volume inside the container changes with a difference volume being at least the volume defined by the central lumen, any liquid agent will be forced through the entire length of the lumen to rinse and/or disinfect the lumen. Advantageously, the difference volume is larger) than the volume defined by the lumen (e.g. at least 110%, at least 125% or at least 150% of the volume defined by the lumen) such that some liquid agent also reaches the distal end cavity.

Additionally or alternatively, the distance along the axial direction which the urinary catheter can be displaced is at least 0.3 cm, at least 0.5 cm or at least 1 cm. With a sufficiently large displacement distance it is ensured that sufficient liquid agent is forced up into the distal end cavity to achieve disinfection.

In some implementations, the catheter shaft of the urinary catheter is coated with a hydrophilic coating.

Intermittent urinary catheters coated with a hydrophilic coating are, as such, known in the art. The hydrophilic coating becomes slippery when wetted with a wetting liquid, which makes insertion and removal of the urinary catheter more comfortable. A reusable hydrophilic urinary catheter is therefore advantageous since this provides convenient catheterization while also reducing the amount of waste produced.

The hydrophilic coating may benefit from re-wetting prior to each use, whereby the liquid agent may comprise a wetting component which wets the hydrophilic urinary catheter. The liquid agent may also help in rinsing the central lumen and the distal end cavity of urinary catheter between uses. The hydrophilic coating may benefit from regeneration to ensure that the hydrophilic and slippery properties of the coating are maintained over time. To this end, the liquid agent may comprise regeneration component.

The liquid agent inside the urinary catheter assembly is hereby adapted for achieving at least one of disinfection, wetting, rinsing and regeneration of the urinary catheter.

The liquid agent may accordingly comprise at least one of a wetting component, a disinfecting component, a regenerative component configured to regenerate the hydrophilic coating, an osmolality increasing component and a diluent component.

In some implementations, the container is flexible.

A benefit with a flexible container is that it may be bent, folded or otherwise deformed alongside the catheter shaft in the storage state to make the urinary catheter assembly easier for users to bring along and making it easier to store in a bag or pocket. However, advantageously the container is still sufficiently rigid to withstand the pressure increase/decrease inside the container when the catheter is displaced axially (while being sealed by the liquid and/or gas tight seal) as the desirable piston effect that forces the liquid agent through the lumen may otherwise be counteracted.

Alternatively, the container is rigid. In one example, the second portion of the container is flexible while the first portion is rigid, wherein the liquid and/or gas tight seal is formed in the first portion. A rigid first portion may make the liquid and/or gas tight seal more reliable and at the same time, a flexible second portion makes it possible to bend the urinary catheter assembly where the catheter shaft is located.

In some implementations, at least one of the distal end cavity and the first portion of the container is provided with at least one marking, indicating an amount of liquid agent present inside the urinary catheter assembly.

The at least one marking may e.g. indicate a minimum level of the liquid agent required for the liquid agent to reach all critical surfaces of the urinary catheter, or a maximum level for the liquid agent where there is a risk for overflow when the urinary catheter is inserted into the container.

In some implementations, the first portion of the container has a larger cross-sectional dimension compared to the second portion of the container, or vice versa.

A larger first portion makes it easier to house the sealing arrangement and/or the holder arrangement inside the container, whereas the second portion of the container (used mainly for housing the catheter shaft) can be made smaller, to make the full assembly more compact in the closed storage state.

In some implementations, at least a portion of the holder arrangement is exposed outside the container when the urinary catheter is inserted into the container and when the lid seals the distal end cavity.

Hereby, since the liquid agent is trapped by the sealing arrangement and the lid closing the distal end cavity, it is possible to have at least a portion of the holder arrangement exposed outside the lid and the container in the storage state. This makes it easier to use the urinary catheter assembly since the user can grab the holder arrangement prior to and during the process of opening the lid and removing the urinary catheter from the container.

In some implementations, the lid is attached to the container with an attachment element.

By attaching the lid to the container the risk of the user misplacing the lid or dropping the lid on the ground is reduced when the lid is opened and the urinary catheter is removed from the container. The attachment element may advantageously be configured such that it is not possible to close the lid if the catheter is not fully inserted into the container and/or configured such that it is not possible to remove the catheter from the container without first removing the lid. This will ensure that the user properly inserts the catheter after each use and that the user opens the lid prior to removing the catheter from the container, which helps ensuring that the above mentioned piston effect is properly exploited during each use.

According to a second aspect of the invention there is provided a method for removing a urinary catheter from a urinary catheter assembly. The urinary catheter assembly comprises a container extending along an axial direction, from an open first portion to a closed second portion and a urinary catheter comprising a catheter shaft with a central lumen and a holder arrangement attached to a distal region of the catheter shaft. The catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen, wherein the urinary catheter is inserted into the container such that a sealing arrangement forms a liquid and/or gas tight seal between the holder arrangement and the container, wherein the sealing arrangement is configured to allow the urinary catheter to be displaced in the axial direction while maintaining the liquid and/or gas tight seal. The urinary catheter assembly further comprises a lid, sealing the distal end cavity of the holder arrangement. The method comprises removing the lid so as to open the distal end cavity, pulling the urinary catheter by the holder arrangement out of the container, while the sealing arrangement forms a liquid and/or gas tight seal, and removing the catheter from the container.

According to a third aspect of the invention there is provided a method for inserting a urinary catheter into a container to form a closed urinary catheter assembly. The urinary catheter assembly comprising a container extending along an axial direction, from an open first portion to a closed second portion and a urinary catheter comprising a catheter shaft with a central lumen and a holder arrangement attached to a distal region of the catheter shaft. The catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen. The urinary catheter assembly further comprising a sealing arrangement configured to form a liquid and/or gas tight seal between the holder arrangement and the container, wherein the sealing arrangement is configured to allow the urinary catheter to be displaced in the axial direction while maintaining the liquid and/or gas tight seal and a lid configured to seal the distal end cavity of the holder arrangement. The method comprising inserting the urinary catheter into the container with the catheter shaft first, pushing the urinary catheter by the holder arrangement into the container, while the sealing arrangement forms a liquid and/or gas tight seal, and attaching the lid to the distal end cavity.

Optionally, the second and/or third aspect of the invention may further comprise the step of verifying that there is a sufficient amount of liquid agent in the container (for disinfection and/or wetting etc.).

During withdrawal of the urinary catheter this may be accomplished prior to pulling the urinary catheter by the holder arrangement out of the container by looking onto the distal end cavity and seeing the liquid agent. During insertion of the urinary catheter this may be accomplished prior to inserting the urinary catheter into the container by looking into the container and seeing the liquid agent and/or prior to attaching the lid to the distal end cavity by looking into the distal end cavity and seeing the liquid agent.

Additionally or alternatively, the container may be transparent, or at least a portion of the container is transparent to form a see-through window, allowing the liquid agent to be seen from the outside of the container. By further providing a marking visible through a transparent portion of the container it is possible to verify the amount of liquid agent inside the assembly at any time, regardless of the catheter being removed from the container or inserted into the container. The marking may e.g. indicate a recommended minimum level of liquid agent allowing the user to easily determine when to refill the liquid agent.

The inside of the container and/or the distal end cavity may be provided with markings indicating a recommended level of liquid agent. If no liquid agent is visible, or does not reach the marking, the method of the second and/or third aspect may comprise the step of refilling or replacing the liquid agent.

### BRIEF DESCRIPTION OF THE DRAWINGS

The present invention will be described in more detail with reference to the appended drawings, showing currently preferred embodiments of the invention.
Figure 1 illustrates a urinary catheter assembly according to some implementations.
Figure 2 illustrates a urinary catheter with a holder arrangement according to some implementations.
Figure 3 illustrates a container, for housing a urinary catheter with a holder arrangement, according to some implementations.
Figures 4a-g illustrate how a urinary catheter is inserted and subsequently withdrawn from the container between uses.
Figures 4h-i are flowcharts illustrating a method for withdrawing and inserting a urinary catheter into a container, according to some implementations.
Figures 5a-b illustrate an alternative holder arrangement comprising threads for threaded engagement with the container.
Figures 6a-c illustrate another alternative holder arrangement comprising two releasably connected parts.
Figures 7a-c illustrate yet another alternative holder arrangement comprising a sleeve acting as a sealing element, according to some implementations.
Figures 8a-c illustrate an alternative sealing arrangement comprising a sealing element arranged on the container.
Figures 9a-b illustrate an exemplary container for bending or coiling the catheter shaft, according to some implementations.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. It may also be noted, for the sake of clarity, that the dimensions of certain components illustrated in the drawings may differ from the corresponding dimensions in real-life implementations of the invention.

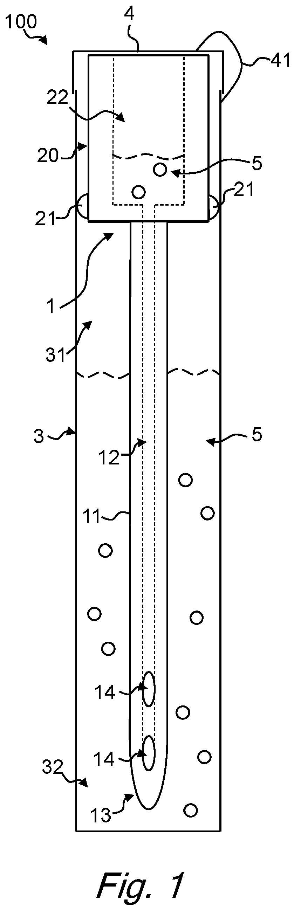

Fig. 1 shows schematically a urinary catheter assembly 100 according to some implementations. The urinary catheter assembly 100 may alternatively be referred to as a reusable urinary catheter assembly since the catheter can be used for catheterization on multiple occasions and put back into the container between uses.

The urinary catheter assembly 100 comprises a container 3, a lid 4 and an, optionally reusable, urinary catheter 1. The container 3 also holds a liquid agent 5. In fig. 1 the urinary catheter 1 is inserted into the container 3 and the lid 4 is attached to the distal end cavity 22, to seal the distal end cavity 22, meaning that the urinary catheter assembly 100 is in its closed storage state. In the closed storage state, the liquid agent 5 is prevented from leaking out and the catheter shaft 11 of the urinary catheter 1 as well as the distal end cavity 22 are protected from the environment. In fig. 1 the urinary catheter assembly 100 is held vertically whereby the liquid agent 5 is collected towards the bottom (i.e. at the second portion 32) of the container 3 and towards the bottom of the distal end cavity 22 while a column of liquid agent 5 extends through the central lumen 12. While the entire catheter shaft 11 is not completely submerged in liquid agent 5 in fig. 1 it is understood that as the user brings the urinary catheter assembly 100 along, the liquid agent 5 will move around inside the container 3 to reach substantially the entire catheter shaft 11 and all surfaces of the distal end cavity 22.

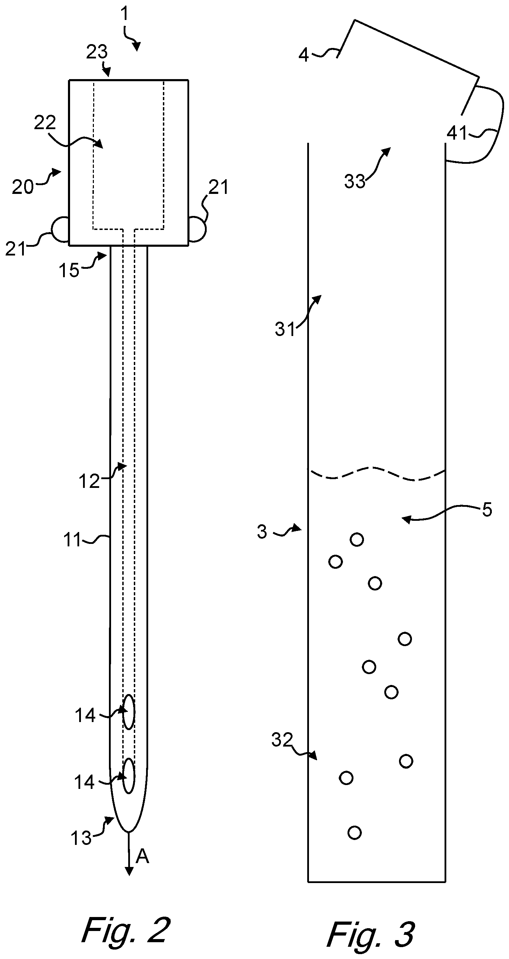

Fig. 2 shows the urinary catheter 1 of the urinary catheter assembly, after it has been removed from the container, according to some implementations. The urinary catheter 1 comprises an elongated catheter shaft 11 extending along an axial direction A. The catheter shaft 11 extends along the axial direction, from a distal end 15 to a proximal insertion end (at the proximal tip 13).

The terms "proximal" and "distal" are in this disclosure used to describe relative position of parts of the urinary catheter 1 with respect to the user when he or she uses the catheter for self-catheterization to drain urine from his or her bladder. The proximal tip 13 of the catheter shaft 11 is inserted first through e.g. the urethra or a stoma of the user to reach the bladder, whereby the tip 13 of the catheter shaft 11 is closer to, i.e. more proximal to, the bladder of the user during self-catheterization whereas the opposite distal end 15 is more distant to, i.e. distal to, the bladder of the user. The terms "proximal" and "distal" may also be used to describe features of the container wherein the proximal end of the container is the closed part of the container that is closest to the proximal tip 13 of the urinary catheter in the closed storage state and the distal end of the container is closer to the holder arrangement 20 and distal end 15 of the urinary catheter 1 in the closed storage state.

As seen in fig. 2, the proximal tip 13 of the catheter shaft 11 may be generally rounded to make insertion of the catheter shaft 11 into e.g. the urethra more comfortable.

The catheter shaft 11 further comprises a central lumen 12 configured to allow urine from the bladder of a user to flow through the catheter shaft 11, through the central lumen 12 into a distal end cavity 22. In fig. 1 and fig. 2, the central lumen 12 and the distal end cavity 22 are illustrated with dashed lines.

To allow urine to enter the central lumen 12 of the catheter shaft 11 from the bladder, the catheter shaft 11 is further provided with one or more drainage openings 14 (often referred to as eyelets) that communicate with the central lumen 12. This allows urine to enter the central lumen 12 of the catheter shaft 11 via the at least one drainage opening 14 when a proximal part of the catheter shaft 11 is inserted into the bladder. Since it typically is only a limited proximal part of the catheter shaft 11 that will enter into the bladder during catheterization, the at least one drainage opening 14 is arranged close to the proximal end of the catheter shaft 11. For example, the at least one drainage opening 14 is arranged within 10 centimeters, or within 5 cm from the proximal end of the catheter shaft 11.

In fig. 2 the catheter shaft 11 is provided with two drainage openings 14, however it is envisaged that the catheter shaft 11 may be provided with a single drainage opening 14 or more than two drainage openings 14, such as ten or more drainage openings 14 or even one hundred or more drainage openings 14, distributed over the proximal portion of the catheter shaft 11. Generally, the total opening area of the drainage openings 14 is around 75 - 200% of the cross-sectional area of the central lumen 12, whereby when a larger number of drainage openings 14 are used each individual drainage opening 14 is generally smaller and vice versa.

The catheter shaft 11 may be made of a plastic material. For example, the catheter shaft 11 is made of a thermoplastic material, e.g. a thermoplastic polymer or thermoplastic elastomer. Additionally or alternatively, it is envisaged that the catheter shaft is made of a material comprising natural fibers such as cellulose fibers as described in European Patent Application no. 21159364.5, hereby incorporated by reference in its entirety.

A hydrophilic coating may be arranged on the catheter shaft 11, e.g. at least over an insertable length thereof. Alternatively, the catheter shaft 11 may be formed by a hydrophilic material. The hydrophilic coating or material provides a very low friction to the catheter shaft 11 when wetted with a wetting liquid.

The distal end 15 of the catheter shaft 11 is connected to a holder arrangement 20. The holder arrangement 20 defines a distal end cavity 22 in (flow) communication with the central lumen 12 of the catheter shaft. The distal end cavity 22 further comprises a cavity opening 23, enabling urine to flow from the bladder, through the at least one drainage opening 14 into the central lumen 12, into the distal end cavity 22 and out via the cavity opening 23. Hereby, a user can hold the catheter by the holder arrangement 20 and insert the catheter shaft 11 into the bladder to drain urine via the distal end cavity 22, without the user coming into contact with the urine.

In some implementations, the holder arrangement 20 is further configured to be connected to a urine collection bag or extension tube, allowing urine to be drained directly into the collection bag or extension tube (leading e.g. into a drain or toilet) via the distal end cavity 22. Accordingly, the holder arrangement 20 may generally be referred to as a "connector" since it resembles a traditional urinary catheter connector that can be connected to e.g. a urine collection bag or extension tube. The holder arrangement 20 may be made as a single part or realized using multiple parts, e.g. with a dedicated connector part resembling a traditional urinary catheter connector and a handling part releasably connected to the connector part, as described below.

However, the holder arrangement 20 according to embodiments of the present invention may form a liquid and/or gas tight seal between the holder arrangement 20 and the container, with the aid of a sealing arrangement. In some examples, a sealing element 21 (e.g. in the form of a gasket) configured to form a liquid and/or gas tight seal with the container is provided on the holder arrangement 20, as described below.

Fig. 3 shows a cross-sectional view of an exemplary container 3. The container comprises a second portion 32 (in which the proximal end of the catheter is housed in the closed storage state) and an opposite first portion 31 (in which the distal end of the urinary catheter is housed in the closed storage state). The container 3 may be substantially rigid, e.g. made of a rigid plastic material or of a composite material comprising natural fibers and a polymer.

Alternatively, the container 3, or at least parts thereof, may be flexible, e.g. made of a thin flexible plastic material allowing the container 3 to e.g. be bent, twisted or coiled. Since the catheter shaft of the urinary catheter also may be flexible, this allows the urinary catheter assembly to be e.g. bent or folded to make it more compact and easier for users to bring along.

To retain any liquid agent and optionally to keep the entire urinary catheter in a closed space, the urinary catheter assembly further comprises a lid 4, configured to at least close the distal end cavity 22 in the storage state. In some implementations, the lid 4 may also form a seal against the container 3 and form a closed space together with the container 3 to fully surround the urinary catheter including the holder arrangement. However, since the sealing arrangement contributes to containing the liquid agent, it is generally not necessary for the lid 4 and the container 3 to form an enclosure around the entire urinary catheter in the storage state. That is, to contain the liquid agent 5 when the catheter is inserted into the container it is sufficient for the lid 4 to close the distal end cavity 22.

The lid 4 is a component which the user will have to remove prior to using the catheter. Hereby, the lid 4 may advantageously be connected to the container via an attachment element 41 (e.g. a piece of string, a strap, a hinge, pivot joint or similar) allowing the lid 4 to be removed and reattached while still ensuring that the user does not misplace the lid 4 after disconnecting it from the distal end cavity and/or the container 3. Since the lid 4 is to be removed and closed each time the catheter is used it is understood that the lid 4 as such can be closed and opened many times.

The attachment element 41 is advantageously configured such that it is not possible to close the lid 4 if the catheter is not fully inserted into the container and/or configured such that it is not possible to remove the catheter 1 from the container 3 without first removing the lid 4. This will ensure that the user properly inserts the catheter 1 after each use (since otherwise it is not possible to close the lid 4) and that the user opens the lid 4 prior to removing the catheter 1 from the container 3. Additionally, to fully utilize the piston effect for pushing liquid agent into the distal end cavity during catheter insertion, and pulling liquid agent out of the distal end cavity during catheter removal, it is beneficial if the lid 4 is attached only after the catheter has been fully inserted and before the catheter is pulled out.

Turning back to fig. 1, the urinary catheter 1 is here shown inside the container 3 wherein the lid 4 has been used to seal the distal end cavity 22 of the holder arrangement 20.

Notably, the sealing arrangement is here realized with a sealing element 21 (here in the form of a sealing gasket surrounding the holder arrangement 20) forming a liquid seal between the inner surface of the container 3 and the holder arrangement 20. Hereby, any liquid agent 5 in the container 3 becomes substantially trapped inside a space defined by the container 3 and the holder arrangement 20, mainly in the second portion 32 of the container 3. It is possible for the liquid agent 5 to enter the central lumen 12 via the drainage openings 14, and by extension reach the distal end cavity 22 via the central lumen 12, but since the lid 4 closes the distal end cavity 22, the liquid agent 5 is prohibited from escaping from the assembly 100.

It is generally advantageous for the liquid agent 5 to reach both the central lumen 12 and the distal end cavity 22, since these parts will come into contact with urine during use and benefit from disinfection or rinsing between uses. On the other hand, the outer surface of the holder arrangement 20 is typically where the user grabs the urinary catheter 1 during use and this surface will generally not come into contact with urine during catheterization. Accordingly, this surface of the holder arrangement 20 does not benefit from disinfection and is advantageously kept dry to make it easier for users to achieve a firm grip.

With the urinary catheter assembly 100 of the present invention, effective disinfection is achieved while the outer surface of the holder arrangement 20 is kept out of contact with the liquid agent 5. To keep the gripping surface of the holder arrangement 20 out of contact with the liquid agent 5, the sealing element 21 may be arranged close to a proximal end of the holder arrangement 20 or at least arranged on a proximal half-portion of the holder arrangement 20.

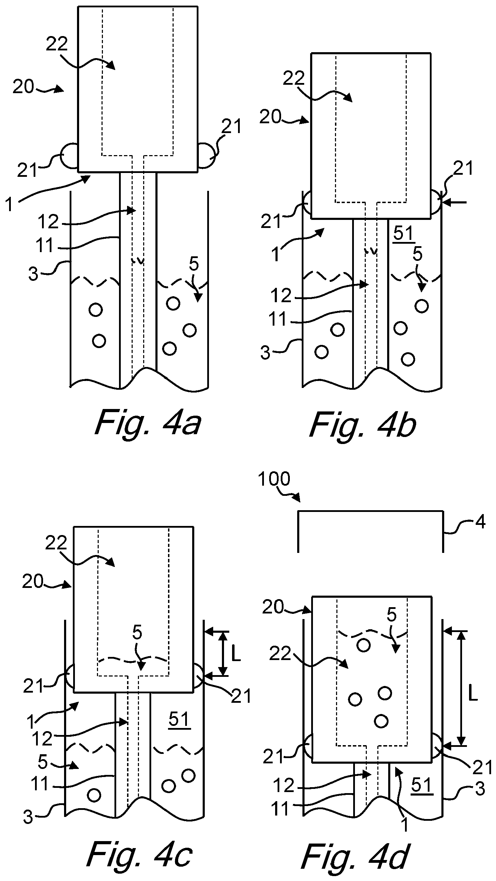

Turning to figs. 4a-g the urinary catheter assembly according to some implementations will now be described in further detail by describing a typical use case. In figs. 4a-g the sealing arrangement is realized with a sealing element 21 in the form of a gasket arranged on the holder arrangement 20 however, as described below, this is merely one out of multiple examples of suitable sealing arrangements envisaged in this disclosure.

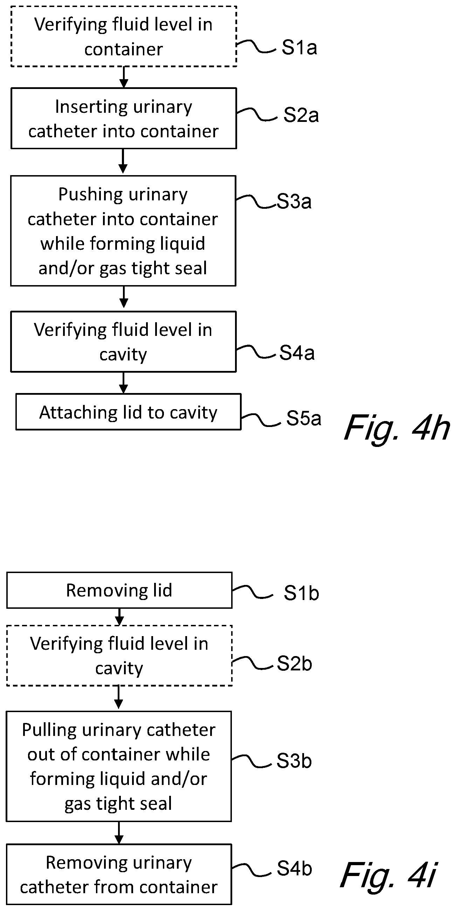

In fig. 4a, the urinary catheter 1 has been used for catheterization whereby the catheter shaft 11, the central lumen 12 and the distal end cavity 22 have all come into contact with urine drained from the bladder of the user. With further reference to the flowchart of fig. 4h, a method for inserting the urinary catheter 1 into the container 3 will now be described.

At step S1a it is verified if the level of the liquid agent 5 inside the container 3 is sufficient. If the container 3 is sufficiently filled with the liquid agent 5 the method goes to step S2a wherein the user inserts the urinary catheter 1, with the catheter shaft 11 first, into the container 3. During step S2a at least a portion of the catheter shaft 11 becomes submerged in the liquid agent 5 (see e.g. fig. 1). Advantageously, the user holds the container 3 substantially vertically at this stage, such that the liquid agent 5 does not pour out.

As the catheter shaft 11 is submerged into the liquid agent 5 the liquid agent 5 enters the central lumen 12 via the drainage openings (see e.g. fig. 1) and may be forced, e.g. by capillary forces, towards the distal end of the central lumen 12.

As the user continues to insert the urinary catheter into container 3 the catheter shaft 11 will progressively displace more and more of the liquid agent 5, whereby the level of the liquid agent 5 rises inside the container and central lumen 12 and air is forced out of the container 3. For example, using Jurin's law for liquid columns with a contact angle of zero degrees, and water as the liquid agent 5, the liquid agent 5 is estimated to rise about 7.8 mm inside a 1.9 mm diameter central lumen.

When the urinary catheter 1 reaches the position shown in fig. 4b the sealing element 21 engages the container 3 and here forms a gas tight seal. At this point, the liquid agent 5 as well as air is prevented from escaping through a space between the holder arrangement 20 and the container 3. Accordingly, when the urinary catheter reaches the position shown in fig. 4b an air pocket 51 is also trapped inside the container 3 alongside the liquid agent 5.

Embodiments are also envisaged where the seal is only liquid tight (and not gas tight) as described below.

The sealing arrangement is further configured to allow the urinary catheter 1 to continue to be displaced axially, an axial distance L, while the gas tight seal is maintained as shown in figs. 4c and 4d. During step S3a the urinary catheter 1 is pushed into the container 3 while the sealing arrangement maintains the gas tight seal. Accordingly, the liquid agent 5 as well as the air pocket 51 is put under pressure which forces the liquid agent 5 into the drainage outlets, through the lumen 12 and out into the distal end cavity 22 so as to reach all critical surfaces in need of e.g. disinfection between subsequent uses of the urinary catheter 1.

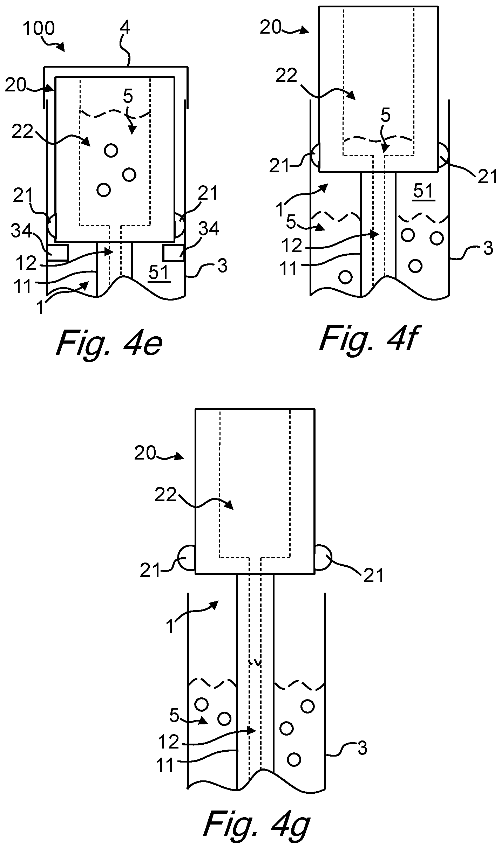

Optionally, a stopping element 34, such as a lip or other types of protrusions is provided on the inner surface of the container as shown in fig. 4e to prevent the urinary catheter 1 from being inserted too far. For example, it is generally desired to prohibit the user from inserting the urinary catheter such that the proximal tip of the catheters shaft 11 comes into contact with closed end of the container 3. The stopping element 34 further ensures that the urinary catheter 1 is inserted the same distance each time, meaning that the amount of liquid agent 5 which is displaced is the same during each insertion of the catheter. Hereby, it is possible to stop too much liquid agent from being forced through the lumen which would pose a risk of overflowing the distal end cavity 22.

The sealing arrangement allows the urinary catheter to act as a piston inside the container 3, to force the liquid agent 5 into the central lumen 12 and the distal end cavity 22. Notably, the liquid agent 5 will still efficiently be forced up to the distal end cavity 22 even if the container 3 has a low level of liquid agent 5, since the trapped air inside the container 3 may force the liquid agent 5 through the drainage openings and the central lumen 12. At the same time, the distal end cavity 22 acts as a reservoir which may store an amount of liquid agent 5 to prevent overflow, should the container 3 be filled with a large amount of liquid agent 5 prior to insertion of the urinary catheter into the container 3.

When the urinary catheter 1 has been inserted into the container 3, as shown in fig. 4d, the method may go to step S4a involving verifying the level of the liquid agent inside the distal end cavity. If the liquid agent 5 is seen inside the distal end cavity 22 the user can be assured that all critical surfaces of the catheter 1 have come into contact with the liquid agent 5.

At step S5a the lid 4 is used to close the distal end cavity 22, trapping the liquid agent 5 in the space between the sealing element 21 and the container 3, inside the central lumen 12 and inside the distal end cavity 22.

Fig. 4e shows the urinary catheter inside the container 3 in the storage state, with the lid 4 closing the distal end cavity 22. In the closed storage state, the outer surface of the holder arrangement 20 is kept out of contact with the liquid agent 5 while at the same time the liquid agent 5 can reach all critical surfaces of the urinary catheter 1 that are in need of disinfection or rinsing. It is noted that the space between the sealing element 21 and the container 3 and the distal end cavity 22 need not be fully filled with the liquid agent since the liquid agent will move around inside these spaces as the user handles the closed urinary catheter assembly. However, without the aforementioned piston effect forcing the liquid agent 5 up to the distal end cavity 22 it may otherwise be difficult to get the liquid 5 to reach the distal end cavity 22 at all.

In some implementations, the user is instructed to e.g. shake the urinary catheter assembly prior to opening and/or after closing the urinary catheter assembly 100 to further ensure that all critical surfaces get disinfected or rinsed by the liquid 5.

Turning to fig. 4f and the flowchart of fig. 4i, a method for removing the catheter 1 from the container 3 will now be described.

At step S1b the lid 4 is opened and the method may then go to step S2b involving verifying the level of the liquid agent inside the distal end cavity 22.

At step S3b the urinary catheter 1 is pulled by the holder arrangement 20 out of the container 3 while the gas tight seal is maintained. This forms a negative pressure contributing to pulling the liquid agent from the distal end cavity 22 into the container 3. Furthermore, gravity may assist in allowing the liquid agent 5 to pour out via the drainage openings into the container 3 for catheters having a central lumen 12 with a sufficiently large cross-sectional area. However, for smaller catheters with a central lumen 12 having a smaller cross-sectional area gravity may not be sufficient to remove the liquid agent 5 from the central lumen 12. Hereby, the negative pressure will be especially advantageous for smaller catheters since it helps pull the liquid agent 5 from the central lumen 12 during removal of the catheter 1 from the container 3.

In fig. 4g the urinary catheter becomes free from the container and the method goes to step S4b involving removing the catheter 1 fully from the container 3. At this stage, the catheter is ready to be used (again) for catheterization.

By repeatedly using the urinary catheter 1 and putting it back into the container 3, the urinary catheter is repeatedly disinfected, rinsed and/or wetted to keep a hydrophilic coating slippery and comfortable to use. However, for each use there is a risk that some of the liquid agent 5 is removed, e.g. flushed away with the urine during catheterization, whereby the amount of liquid agent 5 in the container 3 may decrease for each use. Additionally or alternatively, it is envisaged that the liquid agent 5 is diluted with urine or water (e.g. used to rinse the catheter shaft 11 after use) each time the catheter is used, whereby the amount of liquid in the container 3 remains approximately constant but the wetting and/or disinfection properties of the liquid agent become degraded. Accordingly, the liquid agent 5 may be replaced (or refilled) on a regular basis such as once a day, every second day, twice each week, once a week, or once every two weeks.

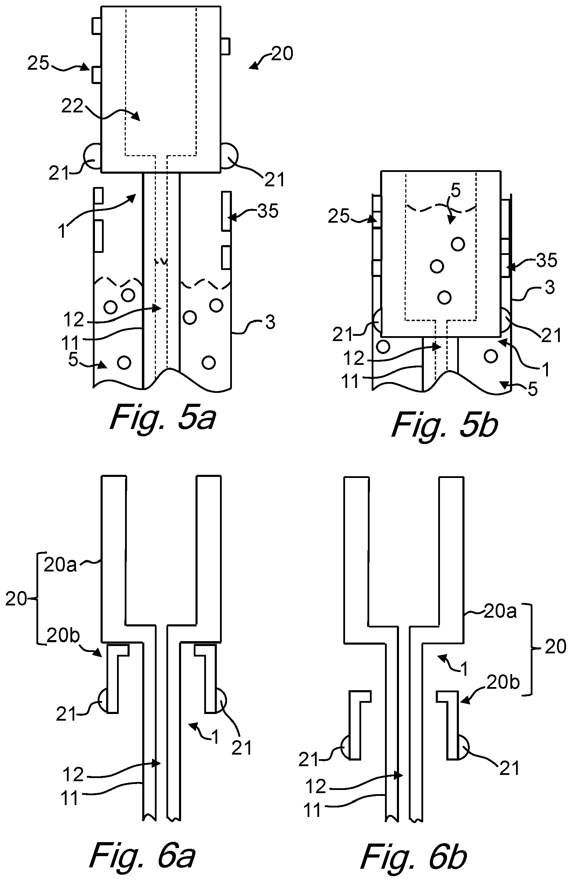

Figs. 5a and 5b depict details of parts of the urinary catheter assembly according to some implementations. The holder arrangement 20 of figs. 5a and 5b is provided with threads 25 configured to threadedly engage matching threads 35 provided on the inner surface of the container 3. In this embodiment, the sealing element 21 is provided on the holder arrangement 20 proximally of the threads 25 such that when the catheter is inserted into the container 3 the sealing element 21 forms a liquid tight seal and subsequently the matching threads 25, 35 engage each other to reliantly hold the urinary catheter 1 inside the container 3. While the seal of this embodiment is liquid tight (but not gas tight) it is envisaged that similar embodiments can be provided with a liquid and gas tight seal.

There are multiple benefits with providing threaded engagement between the holder arrangement 20 and the container 3. Firstly, the threaded engagement is reliable, requiring a twisting force for disengagement, which reduces the risk of the user accidentally removing the urinary catheter or the urinary catheter coming loose from the container 3 when the user carries the urinary catheter assembly in a bag, pocket or similar. Secondly, there is a risk that some user inserts the urinary catheter into the container 3 too rapidly whereby the liquid agent 5 could get ejected via e.g. the distal end cavity 22. This may especially be an issue when the container 3 is filled with an amount of liquid agent corresponding to or exceeding a liquid volume limit. By providing threaded engagement, requiring the user to twist the urinary catheter to reach the desired axial insertion depth into the container, the axial insertion speed of the urinary catheter into the container 3 is limited, reducing the risk of ejecting the liquid agent 5. Another advantage of threaded engagement is that it is possible to define the axial displacement very accurately, which by extension means that the amount of displaced liquid agent 5 forced through the central lumen 12 can be accurately predetermined and will generally be the same each time the catheter 1 is inserted.

While the embodiments of figs. 5a and 5b feature threads 25 arranged on the holder arrangement 20 which are arranged distally of the sealing element 21, it is understood that this is merely exemplary and the container 3 and/or holder arrangement 20 may be adapted to allow the threads 25 to be arranged proximally of the sealing element 21 instead.

In some implementations, the sealing arrangement as such is realized using threads 25, 35. It is envisaged that a liquid tight seal (or even gas tight seal) may be realized using threads 25, 35 provided on the holder arrangement 20 and inner surface of the container 3. Hereby, to insert the urinary catheter 1 into the container and form the liquid tight seal, the user may have to twist the catheter 1 relative to the container 3 a number of revolutions, wherein the threads 25, 35 are configured to provide the liquid tight seal for at least a half, at least one, or at least two revolutions.

The liquid tight seal may still allow gas (such as air) to escape and as seen in fig. 5b no air remains locked inside the container 3 by the sealing arrangement (compare to figs. 4c-d where the gas tight seal traps air inside the container). A gas tight seal is typically also liquid tight, but it is hereby understood that a liquid tight seal is sufficient to obtain the desired piston effect. Accordingly, the sealing arrangement may be liquid tight (but not gas tight) or liquid and gas tight.

For some users, it may be challenging to twist the catheter relative the container 3, whereby the lead of the threads 25, 35 is advantageously adapted such that the user does not need to twist the urinary catheter more than five revolutions to fully insert the urinary catheter into the container 3. As an example, if the urinary catheter is to be axially displaceable at least 1 cm by threaded engagement the lead of the treads is at least 2 mm so as to not require more than five revolutions.

To further facilitate ease of use, the threads may advantageously have multiple start threads, allowing threaded engagement to be started at two, three, four or more rotational orientations of the holder arrangement 20 with respect to the container 3.

Figs. 6a-c illustrate an exemplary holder arrangement 20 according to some implementations. Compared to the holder arrangement 20 of figs. 5a-b the holder arrangement 20 of figs. 6a-c comprises two parts 20a, 20b, namely a connector part 20a and a handling part 20b.

The connector part 20a may be formed as a single piece with the catheter shaft 11 or otherwise fixated to the catheter shaft 11 by means which generally do not allow a user to remove the connector part 20a from the catheter shaft 11. For example, the connector part 20a is attached to the catheter shaft 11 using an adhesive or by means of welding.

The handling part 20b, on the other hand, is a separate piece from the catheter shaft 11 and the connector part 20a. The handling part 20b may comprise an opening through which the catheter shaft 11 can pass and in some implementations, the sealing element 21 is arranged on the handling part 20b. Hereby, the handling part 20b forms a cuff or tube surrounding the catheter shaft 11 allowing the handling part 20b to slide along the catheter shaft 11 and act as a holding element which the user can pinch between his or her fingers so as to make handing of the urinary catheter easier without touching the catheter shaft.

Preferably, the opening in the handling part 20b through which the catheter shaft 11 can pass has an opening diameter generally larger than the outer diameter of the catheter shaft 11. This allows the liquid agent 5 to reach all, or at least substantially all parts of the catheter shaft 11. Engagement between the handling part 20b and the connector part 20a (e.g. threaded engagement or snap lock) may occur at the interface between these two parts 20a, 20b. For example, a distal surface of the handling part 20b engages a proximal surface of the connector part 20a.

Depending on the type of sealing arrangement used (e.g. the positioning of the sealing element 21 on the handling part 20b) it is possible to ensure that at least a portion of the handling part 20b does not contact the liquid agent inside the container 3. In the embodiment of figs. 6a-c the sealing element 21, used to form the sealing arrangement, is in the form of a gasket which surrounds the handling part 20b and by providing the gasket close to a proximal end of the handling part 20b , the distal part of the handling part 20b will generally not get in contact with the liquid agent, whereby the distal part remains dry to touch.

The handling part 20b is further configured to be releasably connected to the connector part 20a. The releasable connection between the handling part 20b and the connector part 20a is advantageously adapted for easy engagement/disengagement by the user, to allow the handling part 20b to be disengaged and slid over the catheter shaft by the user as desired.

As an example, the handling part 20b and the connector part 20a are provided with matching threads or elements forming snap-lock, allowing the handling part 20b to be releasably connected to the connector part 20a. The releasable connection between the handling part 20b and the connector part 20a is advantageously liquid tight (or even gas tight), preventing the liquid agent from escaping between the handling part 20b and the connector part 20a.

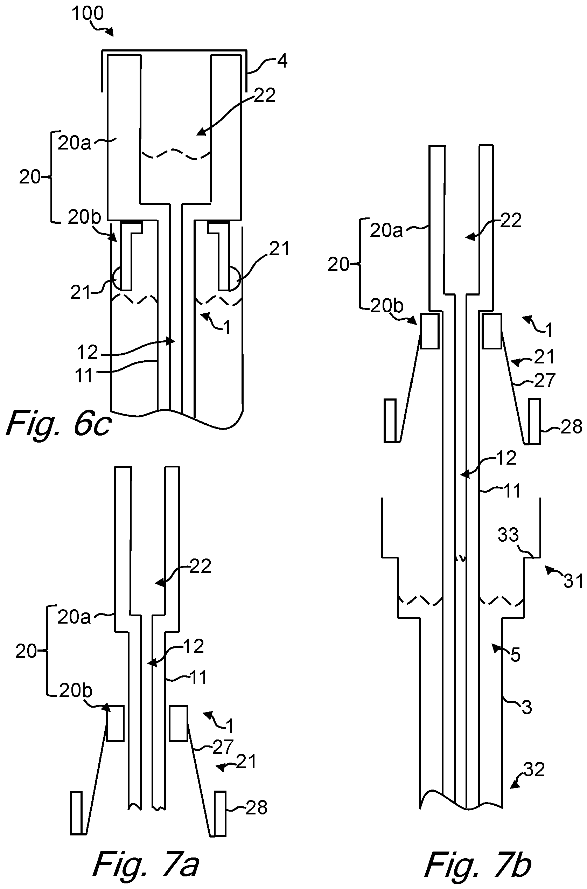

Fig. 6c depicts the handling part 20b being inserted into the container 3. Since the liquid tight seal formed by the sealing element 21 traps the liquid agent, it is possible for at least a distal part of the connector part 20a and optionally also a part of the handling part 20b to be exposed outside the container 3. Hereby, it is noted that at least a portion of the holder arrangement 20 (e.g. the connector part 20a) is exposed outside the container 3 when the urinary catheter is fully inserted. By exposing at least a portion of the connector part 20a it may become easier for the user to withdraw the urinary catheter from the container 3 before use. Even though at least a portion of the connector part 20a is exposed, the liquid agent 5 is still trapped inside the container 3 by the sealing element 21, here provided on the handling part 20b.

The lid 4 may be configured to engage only the connector part 20a to seal the distal end cavity 22 while leaving a portion of the connector part 20a exposed.

In an exemplary use scenario, the user grabs the exposed part of the connector part 20a with a first hand and subsequently removes the lid 4 with the second hand. It is also envisaged that the user may remove the lid 4 while also holding the container 2 with a single hand.

The lid 4 may be loosely attached to the container 3 using an attachment element, allowing the user to drop the lid 4 while the lid 4 is still attached to the container 3. Advantageously, the attachment element is configured such that the lid must be removed to allow the urinary catheter 1 to be pulled out and that the urinary catheter 1 must be inserted fully to allow the lid 4 to be reattached.

At this point, the user may be able to look into the distal end cavity 22 and confirm that some liquid agent 5 is present therein. Liquid agent 5 present in the distal end cavity 22 means that the liquid agent 5 has likely reached all areas of the urinary catheter that are in need of disinfection.

The user may then use their second hand to grab the container 3 and pull and/or twist the urinary catheter with respect to the container 3. During the pulling and/or twisting, the sealing element 21 retains its sealing properties over a portion of the axial displacement of the urinary catheter, whereby a negative pressure builds up and pulls the liquid agent out of the distal end cavity 22 and the central lumen 12. When the urinary catheter becomes free from the container 3, the distal end cavity 22, the central lumen 12, and the outer surface of the catheter shaft 11 may all be wetted and or disinfected by the liquid agent. However, advantageously, a large portion of the liquid agent may have been sucked out of the distal end cavity 22 and the central lumen 12, such that the liquid agent does not drip from the urinary catheter after removal.

At this point, the user holds the urinary catheter by the connector part 20a in the first hand and the container 3 in the second hand. The user may now put away the container 3 and perform catheterization. For example, the user uses their now free second hand to grab the handling part 20b, disengage it from the connector part 20a, and then grip the urinary catheter via the handling part 20b and the connector part 20a while performing catheterization.

To make it easier to put away the container 3 during catheterization, the container 3 or the lid 4 may be provided with a hook or loop allowing it to be hanged on e.g. a clothing hanger. Additionally or alternatively, the lid 4 may be further configured to also close the container 3 when the urinary catheter has been removed therefrom, whereby the lid 4 can be used to close the container, allowing the container 3 to be laid down horizontally without the liquid agent leaking out. It is also envisaged that the second portion 32 of the container 3 is provided with a flat base allowing the container to stand upright.

Turning to figs. 7a-d, cross-sectional views of another exemplary sealing arrangement is depicted. Here, the handling part 20b is releasably connectable to the connector part 20a but it is envisaged that these parts are permanently attached together in some implementations.

The sealing arrangement is here realized with a flexible sleeve 27 attached to the handling part 20b wherein the sleeve 27 extends along a portion of the catheter shaft 11. The flexible sleeve 27 has a narrow end attached to the handling part 20b and a wide end configured for engagement with the container 3. The wide end may be reinforced with a cuff 28 facilitating a better sealing with the container 3. In fig. 7a, the flexible sleeve 27 extends in the proximal direction from the handling part 20b but it is envisaged that it may alternatively extend in the distal direction. Advantageously, the sleeve 27 is made of a flexible material and can be turned inside-out meaning that it can be manipulated to extend either distally or proximally of the handling part 20b.

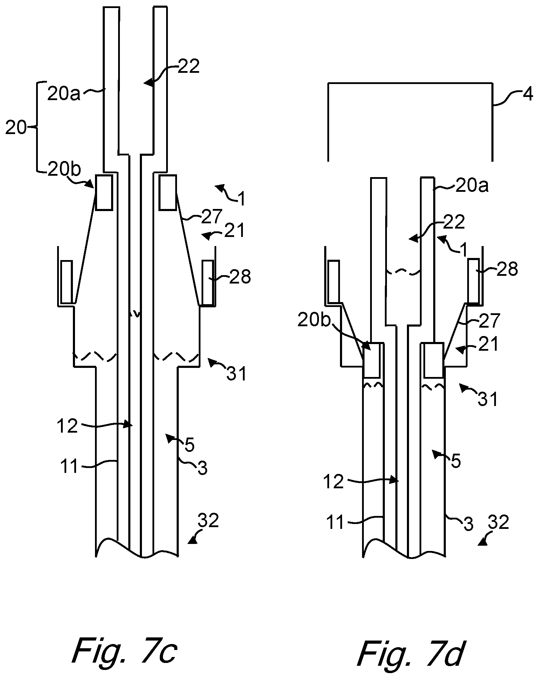

The sealing element in the form of a flexible sleeve 27 may be used together with a container 3 having a varying cross-sectional dimension. For example, in fig. 7b the first portion 31 of the container 3 has a larger cross-sectional dimension and the second portion 32 has a smaller cross-sectional dimension. This forms a ledge 33 for receiving the wide end of the sleeve 27 (or the cuff 28 of the sleeve 27) so as to form the liquid tight seal when the urinary catheter is inserted into the container as shown in fig. 7c.

It is also envisaged that the wide end of the sleeve 27 could engage a rim of the container 3 whereby it is not necessary for the container 3 to have a varying cross-sectional dimension to form the liquid tight seal with the sleeve 27.

Since the sleeve 27 is flexible it is possible to continue to press the urinary catheter into the container 3 even if the wide end or cuff 28 of the sleeve 27 remains static in engagement with ledge 33. Fig. 7c and 7d show how the sleeve 27 can be turned inside-out while the wide end or cuff 28 forms the liquid tight seal. Accordingly, this design allows large axial displacement of the urinary catheter while maintaining the liquid tight seal, which allows the urinary catheter to be properly disinfected between uses even when low levels of disinfection liquid is present in the container. A further benefit with this design is that the wide end of the sleeve 27 rests on the ledge 33, and generally does not slide relative to the container 3 which will reduce the wear on the sleeve 27.

A further benefit with a sealing element 21 in the form of a flexible sleeve 27 which can be turned inside-out, is that the sleeve 27 is easy for users to grip, allowing the sleeve 27 to cooperate with the handling part 20b to make the catheter easier to use. At the same time, since the sleeve is turned inside-out it can expose a large, dry gripping surface when the catheter 1 is removed from the container 3, while at the same time the sleeve 27 is turned inside out in the closed storage state to keep it out of the way of the catheter shaft 11 allowing the catheter shaft to come in contact with the liquid agent 5.

In fig. 7b the sleeve 27 is concave whereby a relatively large dry surface is exposed which the user can grip. Having the sleeve in the concave orientation may be particularly suitable when low levels of liquid agent 5is present in the container 3. When the concave sleeve 27 together with the catheter is inserted into the container, as shown in figs. 7c and 7d, the sleeve 27 is turned inside out to a convex shape. Allowing the catheter shaft 11 to come into contact with the liquid agent 5.

The sealing may be formed by either or both of the cuff 28 and the handling part 20b. As seen in fig. 7d, when the urinary catheter 1 is inserted into the container 3, both the handing part 20b and the cuff 28 may interface with the container 3 and either of both of these parts may participate in forming the sealing arrangement. If the handling part 20b forms part of the sealing arrangement, it is not necessary for the cuff 28 to form a seal, and the engagement between the cuff and the container 3 may be purely for purposes of turning the sleeve 27 inside-out upon insertion.

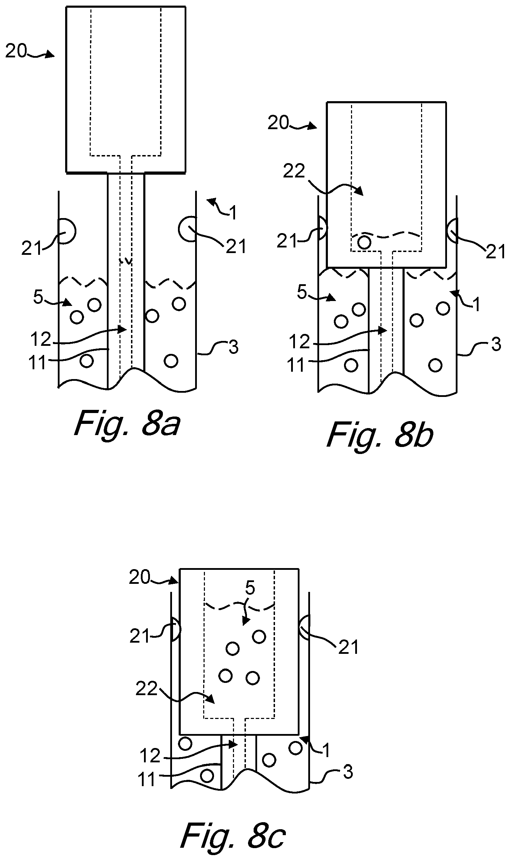

In some implementations, the sealing arrangement comprises a sealing element 21 which is arranged on the inner surface of the container 3 in addition to, or instead of, on the holder arrangement 20. In figs. 8a-c a sealing element 21 in the form of a gasket is attached to the inner surface of container 3. When the urinary catheter is inserted into the container 3 the gasket forms a liquid tight seal against the holder arrangement 20, while still allowing the urinary catheter to be displaced axially while the liquid tight seal is maintained. Accordingly, the sealing arrangements considered herein may involve threaded engagement between the holder arrangement 20 and the container 3 or a sealing element (such as a gasket or sleeve) provided on at least one of the holder arrangement 20 and container.

While the exemplary container 3 of figs. 1 and 3 is generally elongated so as to allow the urinary catheter to be stored substantially without bending the catheter shaft 1, it is understood that the container 3 may be realized with various shapes that allow the catheter shaft to be stored without bending or even necessitating bending of the catheter shaft 1 when inserting the catheter into the container 3.

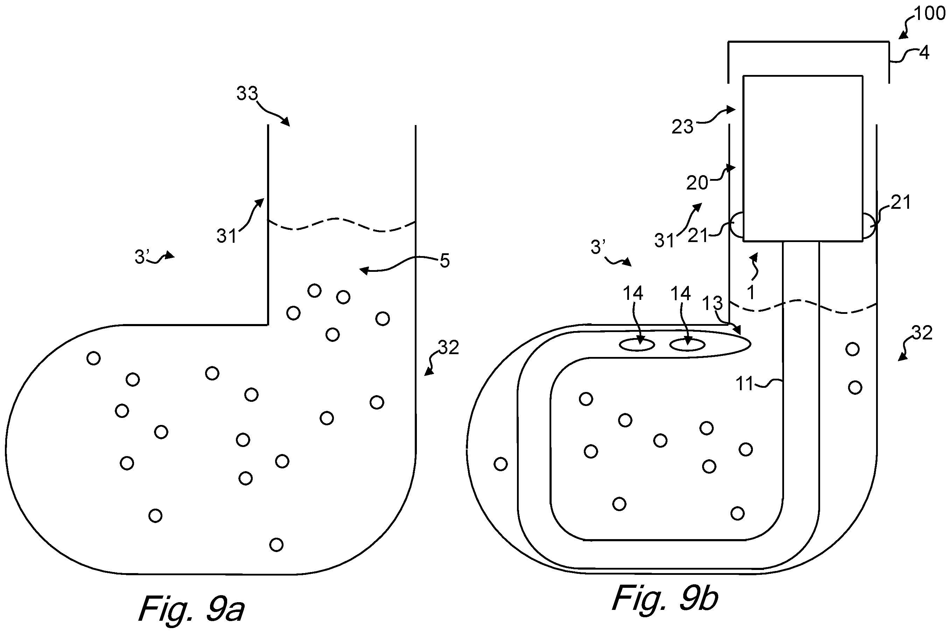

Fig. 9a shows an exemplary container 3' which will force the catheter shaft 11 to bend as the urinary catheter 1 is inserted into the container 3'. This allows the container 3' to be realized with an axial extension which is smaller than the axial extension of the urinary catheter 1 which in turn may lead to a more compact container 3' which is easier to fit in a pocket or bag compared to the generally elongated container 3' of figs. 1 and 3.

The container 3' still has a first portion 31 and a second portion 32 wherein the container opening 33 is provided in the first portion 31 and wherein the first portion 31 is configured to allow the sealing arrangement to form a fluid tight seal when the catheter 1 is inserted into the container 3'.

In fig. 9b the catheter 1 has been inserted into the container 3' with the proximal tip 13 passing through the container opening 33 first. As the catheter 1 is inserted further into the container 3', the catheter shaft 11 is bent or generally coiled by the walls of the container 3' allowing the catheter 1 to be stored in a spatially efficient configuration. Since the catheter shaft 11 of the urinary catheter 1 typically is made of a material exhibiting shape memory and/or elastic resilience, the catheter shaft 11 will revert to its generally straight shape once removed from the container 3'.

While allowing the urinary catheter 1 to be axially displaced, the fluid tight seal formed in first portion 31 of the container still provides the same advantages as described above, such as forcing the liquid agent 5 through the central lumen.

The person skilled in the art realizes that the present invention by no means is limited to the preferred embodiments described above. On the contrary, many modifications and variations are possible within the scope of the appended claims. For example, the various types of sealing elements presented herein (e.g. gasket, threads, or sleeve) may be used separately or combined. Additionally, in the above, the holder arrangement has been described as component which could be realized as a single piece or as two or more pieces and it is understood that the various types of sealing elements may be used with a single-piece or multiple-piece holder arrangement.

In the claims, the word "comprising" does not exclude the presence of other elements or steps than those listed in the claim. The word "a" or "an" preceding an element does not exclude the presence of a plurality of such elements.

## Claims

1. A urinary catheter assembly comprising:
a container extending along an axial direction, from an open first portion to a closed second portion,
a urinary catheter comprising a catheter shaft with a central lumen and a holder arrangement attached to a distal region of the catheter shaft, wherein the catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen,
a sealing arrangement configured to form a liquid and/or gas tight seal between the holder arrangement and the container when the urinary catheter is inserted into the container, wherein the sealing arrangement is further configured to allow the urinary catheter to be displaced in the axial direction while maintaining the liquid and/or gas tight seal, and
a lid, configured to seal the distal end cavity of the holder arrangement.

2. The urinary catheter assembly according to claim 1, wherein the sealing arrangement comprises a sealing element arranged on at least one of the holder arrangements and the container.

3. The urinary catheter assembly according to claim 2, wherein the sealing element comprises a gasket.

4. The urinary catheter assembly according to claim 2 or claim 3, wherein the sealing element comprises a sleeve made of a flexible material, wherein the sleeve comprises a narrow end attached to the holder arrangement and a wide end, wherein at least one of the narrow end and the wide end is configured to form the liquid and/or gas tight seal against the container.

5. The urinary catheter assembly according to any of the preceding claims, wherein the holder arrangement comprises a connector part fixedly attached to the catheter shaft and a handling part configured to be releasably connected to the connector part.

6. The urinary catheter assembly according to claim 5, wherein a sealing element forming part of the sealing arrangement is arranged on the handling part.

7. The urinary catheter assembly according to any of the preceding claims, wherein the holder arrangement is configured for threaded engagement with the first portion of the container.

8. The urinary catheter assembly according to any of the preceding claims wherein the distance along the axial direction which the urinary catheter can be displaced while the liquid and/or gas tight seal is maintained is configured such that a volume inside the container changes with a difference volume being at least a volume defined by the central lumen.

9. The urinary catheter assembly according to any of the preceding claims, wherein the catheter shaft of the urinary catheter is coated with a hydrophilic coating.

10. The urinary catheter assembly according to any of the preceding claims, wherein the container contains a liquid agent, the liquid agent comprising at least one of a disinfecting constituent, a wetting constituent and a regeneration constituent.

11. The urinary catheter assembly according to any of the preceding claims, wherein the container is flexible.

12. The urinary catheter assembly according to any of the preceding claims, wherein at least one of the distal end cavity and the first portion of the container is provided with at least one marking, indicating an amount of liquid agent present inside the urinary catheter assembly.

13. The urinary catheter assembly according to any of the preceding claims, wherein the first portion of the container has a larger cross-sectional dimension with respect to the second portion of the container, or vice versa.

14. The urinary catheter assembly according to any of the preceding claims, wherein at least a portion of the holder arrangement is exposed outside the container when the urinary catheter is inserted into the container and when the lid seals the distal end cavity.

15. The urinary catheter assembly according to any of the preceding claims, wherein the lid is reclosable and attached to the container with an attachment element.

16. The urinary catheter assembly according to any of the preceding claims, wherein the urinary catheter assembly is reusable, and wherein the urinary catheter can be removed from, and reinserted into, the container multiple times and the sealing arrangement is configured to form the liquid and/or gas tight seal each time.

17. A method for removing a urinary catheter from a urinary catheter assembly, the urinary catheter assembly comprising:
a container extending along an axial direction, from an open first portion to a closed second portion,
a urinary catheter comprising a catheter shaft with a central lumen and a holder arrangement attached to a distal region of the catheter shaft, wherein the catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen, wherein the urinary catheter is inserted into the container such that a sealing arrangement forms a liquid and/or gas tight seal between the holder arrangement and the container, wherein the sealing arrangement is configured to allow the urinary catheter to be displaced in the axial direction while maintaining the liquid and/or gas tight seal, and
a lid, sealing the distal end cavity of the holder arrangement, the method comprising:
removing the lid so as to open the distal end cavity,
pulling the urinary catheter by the holder arrangement out of the container, while the sealing arrangement forms a liquid and/or gas tight seal, and
removing the catheter from the container.

18. A method for inserting a urinary catheter into a container to form a closed urinary catheter assembly, the closed urinary catheter assembly comprising:
a container extending along an axial direction, from an open first portion to a closed second portion,
a urinary catheter comprising a catheter shaft with a central lumen and a holder arrangement attached to a distal region of the catheter shaft, wherein the catheter shaft is provided with at least one drainage opening in a proximal region, opposite to the distal region of the catheter shaft, wherein the at least one drainage opening communicates with the central lumen and wherein the holder arrangement comprises a distal end cavity communicating with the central lumen,
a sealing arrangement configured to form a liquid and/or gas tight seal between the holder arrangement and the container, wherein the sealing arrangement is configured to allow the urinary catheter to be displaced in the axial direction while maintaining the liquid and/or gas tight seal, and
a lid configured to seal the distal end cavity of the holder arrangement, the method comprising:
inserting the urinary catheter into the container with the catheter shaft first,
pushing the urinary catheter by the holder arrangement into the container, while the sealing arrangement forms a liquid and/or gas tight seal, and
attaching the lid to the distal end cavity.
